Europäisches Patentamt

European Patent Office

Office européen des brevets

(11) Numéro de publication : **0 317 483 B1**

# (12) FASCICULE DE BREVET EUROPEEN

(45) Date de publication du fascicule du brevet :
**29.04.92 Bulletin 92/18**

(51) Int. Cl.5 : **G01M 3/18,** G01N 27/20,
G01N 29/04, A61F 6/04

(21) Numéro de dépôt : **88500099.2**

(22) Date de dépôt : **26.10.88**

(54) **Méthode pour déterminer la fiabilité des préservatifs masculins.**

(30) Priorité : **30.10.87 ES 8703116**

(43) Date de publication de la demande :
**24.05.89 Bulletin 89/21**

(45) Mention de la délivrance du brevet :
**29.04.92 Bulletin 92/18**

(84) Etats contractants désignés :
**AT BE CH DE FR GB GR IT LI LU NL SE**

(56) Documents cités :
**DE-C- 940 551**
**FR-A- 2 347 684**
**US-A- 2 244 591**
**US-A- 2 474 638**

(56) Documents cités :
**US-A- 2 503 803**
**US-A- 3 621 705**
**PATENT ABSTRACTS OF JAPAN, vol. 7, no.
220 (P-226)[1365], 30 septembre 1983; & JP-
A-58 111 749 (FUJI LATEX K.K.) 02-07-1983**

(73) Titulaire : **DISTREX IBERICA S.A.**
**Tuset n.19**
**E-08006 Barcelona (ES)**

(72) Inventeur : **Torres Ibanez, José**
**Tuset n. 19**
**E-08006 Barcelona (ES)**

(74) Mandataire : **Manresa Val, Manuel**
**Gerona n. 34**
**E-08010 Barcelona (ES)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne une méthode pour déterminer la fiabilité des préservatifs masculins, faisant l'apport de divers avantages qui seront exposés ci-après.

Les matériaux élastomères, tels que le caoutchouc, le latex, etc. vulcanisés ou polymérisés en général, ont des propriétés élastiques, ils sont déformables et peuvent être adoptés ajustés autour des corps où ils sont appliqués, avec de faibles épaisseurs de paroi. Pour ce ils sont utilisés pour la fabrication des préservatifs ou condoms, en constituant une house mince, souple et imperméable en général, qui est appliquée sur le pénis en érection pour empêcher que le sperme entre dans le vagin durant les relations sexuelles et ils sont également utilisés pour éviter la transmission sexuelle de certaines maladies et pour recueillir le sperme aux effets cliniques (analyse, etc.).

Conventionnellement, les préservatifs peuvent présenter un dépôt pour le sperme à son extrémité fermée et ont normalement, un bourrelet de renfort à son embouchure ouverte, pouvant être transparents, translucides, opaques ou rougeâtres, avec leur surface lisse ou rugueuse et secs ou lubrifiés.

Par ailleurs, les préservatifs doivent être exempts d'impuretés, spécialement de substances nuisibles pour la santé.

Cependant, ces matériaux, par leur propre nature (composition et structure ou disposition moléculaire, basiquement), si bien ils permettent leur facile moulage - en général par immersion (plus d'une fois si cela intéresse) de moules correspondants dans un bain de, par exemple, latex naturel avec des stabilisateurs, antioxygènes, réticulateurs, accélérateurs, etc. et ultérieur séchage (par exemple avec de l'air chaud), avec une polumérisation finale (accélérée par la chaleur, le cas échéant) et ultérieur lavage et emballage, avec l'addition de lubrifiants si cela intéresse - donnant lieu à des lots de préservatifs peu homogènes, avec une grande dispersion notamment quant aux caractéristiques physiques et même avec des pores et/ou des entailles affectant son imperméabilité et, pour ce, son degré de fiabilité (ces pores et entailles peuvent être produits para la présence de bulles dans les bains du latex ou par des contractions non souhaitées durant le séchage et/ou la polymérisation). Pour déterminer la fiabilité des préservatifs, on a proposé et utilisé divers essais de contrôle de qualité, y inclus les essais destructifs, qui proviennent d'autres domaines industriels, tels que le textile, le papier et les pneumatiques pour véhicules.

Parmi ces essais on peut citer: celui de résistance à la traction et à l'allongement minimum de rupture, qui est opéré en coupant un morceau ou une éprovette de préservatif à essayer, qui est étiré jusqu'au point de rupture et on mesure la résistance à la traction et à l'allongement dans ladite rupture; celui de volume et de pression d'explosion, dans cet essai on gonfle avec de l'air une longueur constante du préservatif et on note le volume et la pression au moment de son explosion, en effectuant le gonflage avec de l'air propre et avec un débit moyen, constant et déterminé; celui de stabilité de la couleur, dans le cas de préservatifs pigmentés, dans lequel on mouille le préservatif à essayer complètement avec de l'eau distillée et on l'enveloppe dans du papier blanc absorbant, en examinant le papier, après une certaine période de temps, pour observer d'éventuelles marques de teinture; celui de stabilité durant le stockage des préservatifs, par vieillissement accéléré, puisque les élastomères ont tendance à se détériorer avec le temps, pour des causes mécaniques, par l'action de la lumière, etc., de sorte que le préservatif correspondant à essayer sera soumis aux essais déjà décrits de résistance à la traction et on observe l'allongement au moment de la rupture, en mesurant les propriétés de traction et/ou la pression et le volume d'explosion des préservatifs, après avoir été conditionnés dans leurs emballages à une température élevée durant un temps déterminé; et celui de détection de trous dans des zones poreuses, qui peut être réalisée en remplissant le préservatif avec un volume d'eau déterminé et en l'examinant afin de détecter toute fuite d'eau à travers sa paroi ou bien on peut le réaliser en se basant sur une méthode de conductibilité, au moyen d'un récipient métallique en fonction d'électrode, plein d'eau et de sel et dans lequel on introduit le préservatif à essayer, qui est également rempli avec une solution saline et à l'intérieur duquel on introduit une autre électrode, en disposant d'une source de courant continu, une résistance et un voltmètre, pouvant ainsi détecter toute fuite qui serait indiquée par le passage de courant dans le circuit électrique.

Lesdits essais sont destructifs en eux-mêmes ou bien il faut toujours détruire le préservatif une fois essayé.

Un essai destructif est égalment connu dans lequel on injecte de l'eau à l'intérieur du préservatif, à débit constant, jusqu'à la rupture dans l'air.

Dans les essais cités déjà connus, où on injecte l'air ou l'eau à l'interieur du préservatif, disposé au sein de l'air, ladite introduction du fluide correspondant est opérée à débit constant, de sorte que les préservatifs conventionnels composés toujours par des macromolécules (tel que le poly-isoprène dans le latex) sont soumis dans cet essai à des efforts croissants, où la vitesse de croissance est constante, il n'est donc pas possible de détecter avec lesdits essais les équilibres méta-stables dans lesquels se trouvent ou peuvent se trouver ses molécules; et par ailleurs, dans l'usage normal des préservatifs, ils sont soumis à des contraintes dont la vitesse de variation n'est pas constante et ces contraintes ne sont pas homogènes, donc, si bien on admet que le risque théorique de rupture des préservatifs est de l'ordre de 0,6%, dans la practique il est de l'ordre de 1 à 8% et encore plus.

La rhéologie étudie, en tant que science, la déformation et la fluidité des substances, en particulier la fluidité non newtonienne et la plastique; et cette science est particulièrement appliquée aux matériaux élastomères avec lesquels on fabrique les préservatifs. On peut citer, comme théories développées dans la rhéologie, les correspondantes au corps élastique idéal ou corps de Hooke, au corps visqueux - d'après la loi de Newton-, à l'équation de Lennard-Jones, au corps visco-élastique étudié par Arridge, en considérant les distorsions ou changements de forme, avec des déplacements dans lesquels on conserve les distances intermoléculaires et dont l'étude donne lieu à des courbes qui montrent des zones ou des moments d'équilibre méta-stables. On peut également citer la classification suivante de mensurations rhéologiques selon les modèles visco-élastiques: statiques, dans lesquelles la contrainte ou la déformation reste constante durant la mensuration de l'essai; transitoires, dans lesquelles la contrainte ou la déformation est appliquée avec une augmentation constante par rapport au temps, c'est-à-dire, avec une vitesse constante; et dynamiques, lorsque la contrainte ou la déformation est appliquée en suivant une fonction du temps, tel que la sinusoïdale ou en dent de scie, et on mesure la variable en sa valeur et en son décalage par rapport au temps.

En outre, on a proposé des modèles visco-élastiques, tel que le dénommé corps de Maxwell, qui est étudié comme un corps de Hooke en série avec un corps de Newton, et il résulte que le latex en polyisoprène se comporte, dans une première approximation, comme un corps de Maxwell, dans lequel les premières contraintes qui lui sont appliquées produisent une réponse élastique (linéaire) et si la charge se maintient, le composant visqueux intervient et, lorsqu'il annule ladite charge, le corps recule comme un corps élastique mais avec une déformation permanente. Une autre théorie, dénommée enveloppente de fractures de Smith, illustre l'effet de l'influence du facteur temps, par l'enveloppante d'une série de fractures dans un diagramme de tensions et de déformations à diverses vitesses de traction, étant expliqué dans ce cas que la zone de rupture fragile correspond à une rupture dans laquelle il n'a pas existé de temps pour que le matériaux se détende ou s'écoule à l'intérieur, à cause des vitesses relativement grandes d'essai.

Les principaux critères rhéologiques de la rupture des matériaux en question sont basés en ce que la rupture peut se produire: par une séparation moléculaire, lorsque le potentiel de Lennard-Jones est dépassé, la rupture se produisant par séparation physique entre deux molécules, lorsque les forces intermoléculaires ou de Van der Waals qui les unit sont dépassées; par dépassement de la limite visco-élastique, selon des études effectués par Kaelbe; et par la théorie de la propagation de fentes, étudiée par Griffith, en considérant que la rupture des matériaux est due aux imperfections (fentes ou creux) des macromolécules, en considérant sa vitesse de propagation face aux efforts de l'essai.

On a démontré expérimentalement que, pour surmonter les inconvénients propres aux essais déjà connus et en tenant compte des diverses théories rhéologiques exposées, les essais dynamiques sont fondamentaux pour éliminer les équilibres méta-stables, qui peuvent être considérés comme de véritables engorgements moléculaires, lesquels laissent d'être stables avec une vibration moléculaire ou avec le temps, à part qu'on a fait des expériences avec un modèle analogique et le plus proche possible à l'utilisation ou l'usage réel des préservatifs.

A cet effet, il est proposé une nouvelle méthode industrielle pour déterminer la fiabilité des préservatifs masculins, une méthode qui est caractérisée en ce qu'on plonge le préservatif au sein d'un liquide contenu dans un récipient convenable, en ce qu'on injecte un liquide à impulsions dans le préservatif et en ce que l'on provoque des ondes de choc qui arrivent à déchirer le préservatif peu fiable. On juge intéressant un cas de réalisation de ladite méthode, caractérisée en ce qu'on introduit, complémentairement dans le préservatif, une électrode branchée à une source d'alimentation électrique, à son tour branchée à un groupe en parallèle, composé par un voltmètre approprié et à une résistance, un groupe finalement branché à une autre électrode introduite, le cas échéant, dans le liquide du récipient; et en ce qu'on détecte l'existence de trous et/ou de zones poreuses dans le préservatif et en ce que sa rupture se produit.

Par l'application de la méthode objet de l'invention, on traite les préservatifs comme des corps qui ne sont pas cristallins, comme c'est en réalité le cas, dans un essai industriel dynamique qui détecte et élimine les préservatifs non idoines pour leur usage, de sorte que les ondes de choc provoquées déchirent le préservatif peu fiable, car sa pellicule est inestable et même parce que son matériau a des équilibres méta-stables, qui ne se déchirerait pas dans les essais déjà connus et qui ne montrerait pas ses défauts dans ces essais, étant considéré, selon ceux-ci, comme des produits acceptables.

L'application de la méthode objet de l'invention donne lieu aux avantages déjà exposés, en relation avec les essais et méthodes déjà connus, en plus d'autres avantages qu'un expert en la matière déduira facilement, un exemple de réalisation de ladite méthode étant décrit ci-après, pour rendre plus facile la compréhension des caractéristiques exposés auparavant, en portant à la connaissance en même temps divers détails de son exécution.

On dispose un récipient en matériau isolant, tel qu'un matériau plastique, qui est rempli d'eau dans laquelle on dissout une certaine quantité de sel pour faciliter sa conductibilité. Sur un support approprié on monte le préservatif à traiter, qui reste suspendu verticalement vers le bas dudit support, que l'on dispose et fixe sur le récipient et de sorte que le préservatif reste convenablement submergé au sein du liquide dudit récipient, au sein duquel on introduit une électrode et, en traversant le support on introduit une autre électrode à l'intérieur du creux du préservatif suspendu dudit support, en branchant la dernière électrode à une source d'énergie électrique à basse tension, par exemple environ 10 volts de courant alterne (quoique qu'il pourrait également être du courant continu, mais il est moins efficace) et, à son tour, ladite source est branchée à un groupe composé par une résistance et un voltmètre montés en parallèle, ledit groupe est branché, à son tour, à l'électrode submergé directement au sein du liquide contenu dans le récipient.

L'installation décrite mise en marche, on procède à injecter un liquide, tel que l'eau, à l'intérieur du préservatif, et il est caractéristique que ladite injection se fasse par pulsations ou par impulsions, de préférence sous forme de dent de scie, quoique pas exclusivement, en provocant ainsi des ondes de choc qui arrivent à déchirer le préservatif peu fiable ou qui a des défauts et en détectant, en même temps, des trous ou des pores éventuels dans le préservatif à travers l'indication de la tension que peut signaler le voltmètre, indicative du passage d'un certain courant entre les deux électrodes et par la résistance en parallèle avec le voltmètre, ce qui représente un passage de courant à travers la paroi du préservatif et, donc, un trou ou plusieurs et/ou un pore ou plusieurs dans celui-ci, qui le rend inutilisable, le lot auquel appartient ledit préservatif étant donc rejetable et il faudra le détruire.

Si on utilise un recipient métallique, son propre corps peut exercer la fonction de l'électrode submergé au sein du liquide du récipient qu'il remplacera.

Il faut observer que la méthode décrite représente la rupture seulement des préservatifs peu fiables ou défectueux, même dans le cas où on n'aurait pas détecté de trous, de fentes ou de pores dans les préservatifs traités, de sorte qu'il faudra refuser les lots dont le préservatif représentant ou préservatifs en fonction d'éprouvette serait arrivé à se déchirer durant le traitement avec la méthode décrite.

Dans le cas où on utiliserait des préservatifs en vrac ou en grandes quantités, on appliquera la méthode décrite aux échantillons déterminés statistiquement de chaque lot, à part d'appliquer d'autres essais conventionnels, qui ont été décrits auparavant, en déterminant ainsi, par l'application de la méthode décrite, les échantillons refusés et ceux acceptés, ces derniers servant comme étalon pour la fabrication de séries de préservatifs qui, une fois reçus, sont à nouveau traités selon la méthode objet de l'invention, en répétant également les essais conventionnels jugés intéressants, en finissant le processus de fabrication en enroulant les préservatifs, en les lubrifiant, par exemple avec des huiles de silicone, un ensachage hermétique par unités et un emballage en boîtes.

Avant de procéder à l'envoi d'une quantité importante de préservatifs ensachés, on effectue encore une fois le traitement en suivant la méthode décrite, à part d'autres essais conventionnels obligatoires (tels que l'essai de stabilité durant le stockage par vieillissement accéléré, déjà décrit).

Il faut constater que, dans la réalisation de l'objet de l'invention, on pourra appliquer toutes les variantes de détail que l'expérience et la pratique puissent conseiller, en particulier quant aux phases ou opérations complémentaires et autres circonstances de caractère accessoire, et on pourra également introduire toutes les modifications de détail résultant compatibles avec l'essentialité de la méthode, définie dans la revendication première, car tout cela reste compris dans l'esprit des revendications suivantes.

## Revendications

1. Méthode pour déterminer la fiabilité de préservatifs masculins, caractérisée en ce qu'elle consiste à submerger le préservatif au sein d'un liquide contenu dans un récipient et à injecter à impulsions un liquide dans le préservatif provoquant des ondes de choc qui arrivent à déchirer le préservatif peu fiable.

2. Méthode pour déterminer la fiabilité des préservatifs masculins, selon la revendication 1 caractérisée, en ce qu'on introduit une première électrode dans le préservatif, ladite électrode étant branchée à une source d'alimentation électrique, branchée à son tour à un groupe en parallèle, composé par un voltmètre et une résistance, en ce qu'on introduit une deuxième électrode, branchée audit groupe parallèle, dans le liquide du récipient; et en ce que l'on détecte la présence de trous, de zones poreuses dans le préservatif et/ou la rupture du préservatif.

## Patentansprüche

1. Methode zur Bestimmung der Zuverlässigkeit von Präservativen für Männer, dadurch gekennzeichnet, dass das Präservativ in einen mit Flüssigkeit gefüllten Behälter getaucht und dass stossartig Flüssigkeit in das Präservativ gespritzt, wodurch die Stosswellen die wenig zuverlässigen Präservative zerreissen.

2. Methode zur Bestimmung der Zuverlässigkeit von Präservativen für Männer nach Anspruch 1, dadurch gekennzeichnet, dass eine erste Elektrode in das Präservativ eingeführt wird, wobei diese an eine elektrische Quelle angeschlossen ist, die gleichzeitig an eine parallele Gruppe angeschlossen ist, welche sich aus einem Voltmeter und einer Resistenz zusammensetzt, da eine zweite, an besagte parallele Gruppe im Behälter angeschlossene zweite Elektrode eingeführt wird, wodurch das Vorhandensein von Löchern, porösen Zonen im Präservativ und/oder Risse festgestellt werden kann.

**Claims**

1. Method to determine the reliability of condoms characterized in that it consists in sinking the condom in a liquid contained in a container and to inject by impulsions a liquid into the condom, provoking a shock wave that bursts the little reliable condom.

2. Method to determine the reliability of condoms according to claim 1 characterized in that a first electrode is introduced into the condom, said electrode being connected to a power supply in turn connected to a shunt unit composed of a voltmeter and a resistor, in that a second electrode connected to said shunt unit is introduced in the liquid of the container and in that the existence of holes, porous areas and/or the bursting of the condom are detected.